# EUROPÄISCHE PATENTSCHRIFT

(11) **EP 0 575 878 B1**
(45) Veröffentlichungstag und Bekanntmachung des Hinweises auf die Patenterteilung: **10.04.1996**
(21) Anmeldenummer: 93109600.2
(22) Anmeldetag: 16.06.1993
(51) Int. Cl.: A61B 17/34, A61M 25/06

(54) **Anästhesiebesteck**
Set of instruments for anaesthesia
Trousse pour l'anesthésie

(30) Priorität: 24.06.1992 DE 9208414 U
(43) Veröffentlichungstag der Anmeldung: 29.12.1993
(73) Patentinhaber: Haindl, Hans, Dr., D-30974 Wennigsen (DE)
(72) Erfinder: Haindl, Hans, Dr., D-30974 Wennigsen (DE)
(74) Vertreter: Leine, Sigurd, Dipl.-Ing.

(56) Entgegenhaltungen:
- EP-A- 0 362 494
- WO-A-83/03189
- WO-A-89/00436
- DE-C- 3 922 406
- US-A- 5 163 901

## Beschreibung

Die Erfindung bezieht sich auf ein Anästhesiebesteck, bestehend aus einer Epiduralkanüle mit angeschliffener Öffnung, einem dazugehörigen Epiduralkatheter, der durch diese Kanüle hindurchgeführt werden kann und einer Spinalkanüle, die länger und dünner ist als die Epiduralkanüle.

Die Spinalanästhesie und die Epiduralanästhesie sind zwei Verfahren der Regionalanästhesie, die im wesentlichen bei Operationen der unteren Extremität und der Beckenorgane benutzt werden. Beide haben ihre spezifischen Vor- und Nachteile, und es liegt bei bestimmten Indikationen nahe, diese zu kombinieren. Hierzu ist aus der DE 3 922 406 ein Besteck bekannt, bei dem die Epiduralkanüle, deren Schliff seitwärts gebogen ist, noch ein zweites Loch in der Verlängerung ihrer Mittelachse an ihrer Spitze hat. Durch dieses Loch kann eine Spinalkanüle hindurchgeschoben werden, um vor dem Legen des Epiduralkatheters eine Spinalanästhesie zu setzen.

Dieses Verfahren hat sich bewährt, hat aber in der Hand des Ungeübten einen Nachteil. Die korrekte Lage des Epiduralkatheters wird normalerweie mit einer Testdosis Lokal-anesthetikum geprüft. Es wird die Dosis, die einer Spinal-anästhesie entspräche, in den Epiduralkatheter injiziert. Wenn dieser, was manchmal vorkommen kann, fälschlicherweise subdural liegt, kommt es zu einer Spinalanästhesie. Eine unerkannte subdurale Lage kann zu lebensbedrohlichen Komplikationen für den Patienten führen. Bei dem erwähnten Besteck ist diese Kontrolle nicht mehr möglich, da bereits vor dem Schieben des Epiduralkatheters eine Spinalanästhesie gesetzt worden ist. Dies ist ein Nachteil des in der DE 3 922 406 beschriebenen Bestecks.

Der erfinderische Gedanke des nunmehr beschriebenen Anästhesiebestecks besteht darin, daß die Kombination der beiden Verfahren so erfolgt, daß zunächst die Lage des Katheters geprüft werden kann und erst dann die Spinalanästhesie verabreicht wird.

Ein Anästhesiebesteck gemäß dem Oberbegriff des unabhängigen Anspruchs 1, mit dem dieses möglich ist, ist bereits aus der US 4 808 157 und 4 958 901 (korrespondiert mit WO-A-8900436) bekannt. Hierbei handelt es sich um eine Epiduralkanüle mit 2 Lumina, durch die völlig unabhängig voneinander der Epiduralkatheter und die Spinalkanüle geschoben werden können. Diese Kanülen sind aber extrem teuer, da sie sehr aufwendig herzustellen sind, und sie haben einen durch die zwei Lumina bedingten, großen Durchmesser, der bei der Epiduralanästhesie wegen der daraus folgenden Rückenbeschwerden unerwünscht ist.

Bei der im folgenden beschriebenen Erfindung werden die gewünschten Eigenschaften im wesentlichen durch eine geeignete Abstimmung der Kanülen- und Kathetermaße erreicht.

Das erfinderische Ziel wird gemäß dem Kennzeichen des Anspruchs 1 dadurch erreicht, daß der Durchmesser bzw. Querschnitt der Epiduralkanüle so bemessen ist, daß bei darinliegendem Epiduralkatheter noch ausreichend Platz für das Hindurchschieben einer Spinalkanüle besteht. Durch eine großkalibrige Epiduralkanüle, z. B. der Größe 16 G, wird also ein dünner Katheter, z B. der Größe 20 G, hindurchgeschoben. Es bleibt dann zwischen Kanüle und Katheter ausreichend Platz, um z. B. eine Spinalkanüle der Größe 26 G hindurchzuschieben.

Eine besonders vorteilhafte Ausgestaltungsmöglichkeit besteht gemäß Anspruch 2 darin, daß das Kanülenrohr oval gestaltet ist, so daß Katheter und Spinalkanüle darin Platz haben, ohne daß das Rohr in seiner Gesamtheit zu groß werden muß.

Das Hindurchschieben der Spinalkanüle unter den Schliff bei liegendem Katheter ist in diesem Falle nur möglich mit einer Biegung der Spinalkanüle in der Spitze der Epiduralkanüle. Dies könnte Anlaß zur Besorgnis geben, daß die Spinalkanülenspitze dabei beschädigt wird. Dies läßt sich dadurch verhindern, daß gemäß einer Weiterbildung der Erfindung nach Anspruch 3 in dem Spitzenbereich der Epiduralkanüle ein Loch zum Austritt de Spinalkanüle vorgesehen ist, ähnlich wie dies in der DE 3 922 406 beschrieben ist, aber mit dem Unterschied, wie dies im Anspruch 5 angegeben ist, daß das Loch nicht zentrisch, sondern exzentrisch nahe der Wand der Kanüle angeordnet ist.

Um ein Eindringen von Gewebe in dieses zusätzliche Loch zu vermeiden, kann gemäß Anspruch 10 in vorteilhafter Ausgestaltung ein profilierter Mandrin verwendet werden, der das gesamte Lumen der Kanüle verschließt und damit auch das zusätzliche Loch.

Weitere vorteilhafte Ausgestaltungen der Erfindung sind in den weiteren Ansprüchen angegeben.

Die Erfindung soll nachfolgend anhand der beigefügten Zeichnung näher erläutert werden.

Es zeigt
- Fig. 1-4: die Anwendung des erfindungsgemäßen Anästhesiebesteckes,
- Fig. 5: die Einzelkomponenten des Anästhesiebesteckes,
- Fig. 6a + 6b: zwei verschiedene Ausführungsformen einer Epiduralkanüle mit Katheter und Spinalkanüle im Schnitt,
- Fig. 7: die Anordnung einer Spinalkanüle und eines Katheters in einer herkömmlichen Epiduralkanüle,
- Fig. 8: eine Anordnung einer Spinalkanüle und eines Katheters in einer anderen Ausführungsform der Epiduralkanüle,
- Fig. 9 + 10: besondere Ausführungsformen von Luerkegeln am Ansatz einer Spinalkanüle.

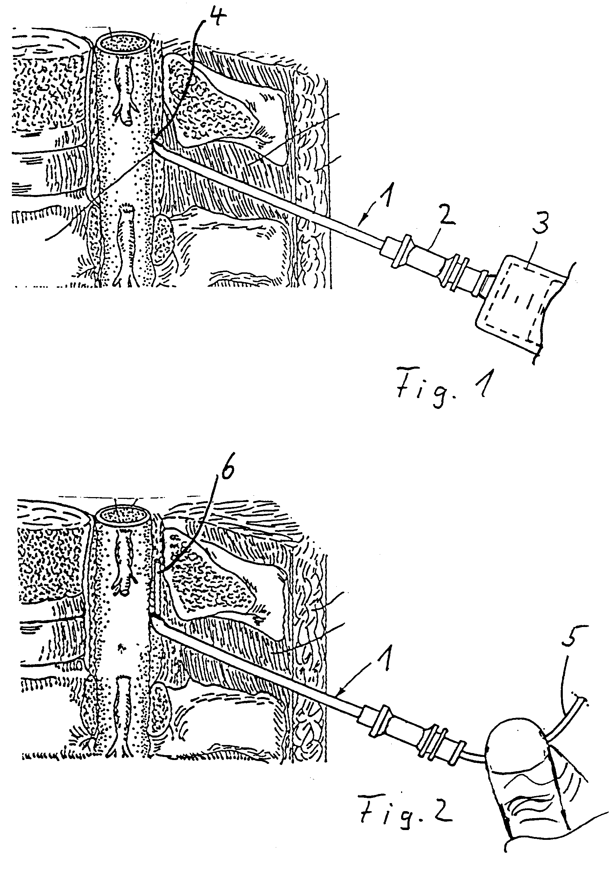

Fig. 1 zeigt die Epiduralkanüle 1 mit dem Ansatz 2 und einer aufgesetzten Spritze 3, mit der in üblicher Weise der Epiduralraum 4 punktiert wird. Nach der Punktion wird die Spritze abgenommen und, wie in Fig. 2 gezeigt, der Katheter 5 eingeschoben, bis dessen Spitze 6 einige Zentimeter weit im Epiduralraum liegt. Danach wird über den Epiduralkatheter die übliche Testdosis zur Kontrolle der korrekten Lage gegeben.

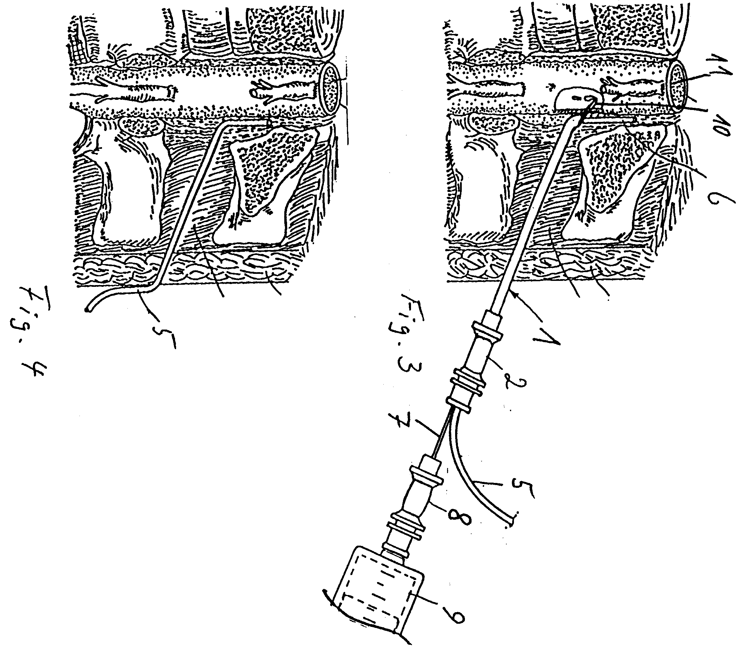

Nun wird, wie Fig. 3 zeigt, eine Spinalkanüle 7, ebenfalls mit einem Ansatz 8 und einer aufgesetzten Spritze 9, neben dem Epiduralkatheter 5 durch die Epiduralkanüle 1 geschoben. Nach dem Austritt der Spinalkanülenspitze 10 perforiert diese die harte Hirnhaut und tritt in den subduralen Raum 11 ein. In diesen wird nun das Medikament zur Spinalanästhesie gegeben. Danach wird die Spinalkanüle 7 entfernt, ebenso die Epiduralkanüle 1 und es bleibt zurück der Epiduralkatheter 5, wie in Fig. 4 dargestellt.

Die Fig. 5 zeigt die Komponenten des Systems, die Epiduralkanüle 1 mit ihrem Ansatz 2 und dem sogenannten Touhy-Schliff mit seiner seitlichen Öffnung 12 am vorderen Ende, den Epiduralkatheter 5 und die Spinalkanüle 7 mit ihrem Ansatz 8, der an seinem vorderen Ende mit einem Luerkegel 8' versehen sein kann, damit sich der Ansatz 8 leichter in den Ansatz 2 der Epiduralkanüle 1 einführen läßt. Die Spinalkanüle 7 ist ebenso wie die Epiduralkanüle 1 beim Einstechen mit einem Mandrin verschlossen, dessen Ansatz 13 am Kanülenansatz zu sehen ist.

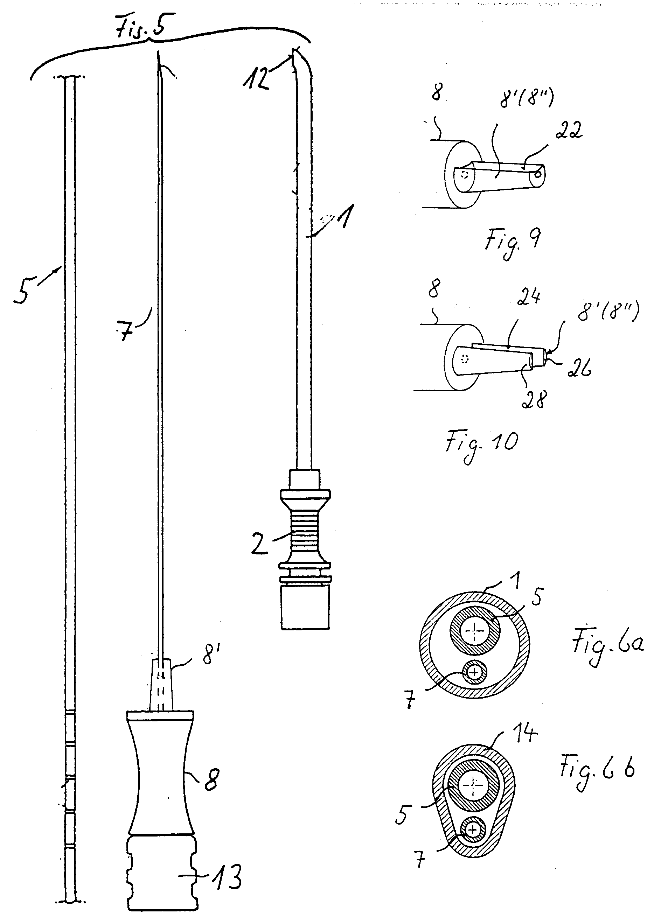

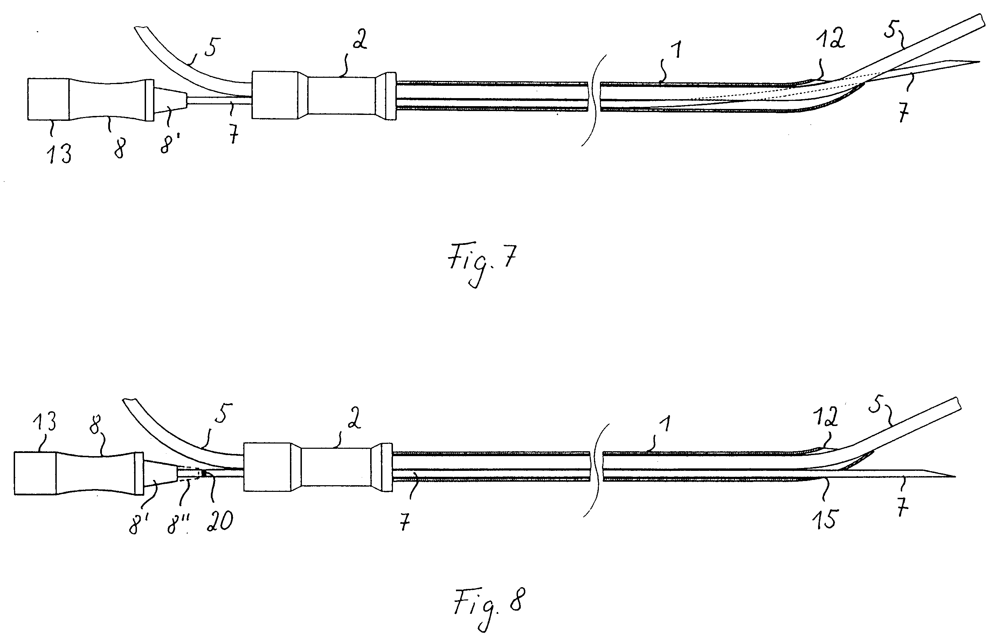

Die Fig. 6a, b zeigen die möglichen Anordnungen von Katheter und Spinalkanüle innerhalb der Epiduralkanüle. Dies kann einmal ein rundes Rohr der Epiduralkanüle 1 sein, in dem sich der Katheter 5 und die Spinalkanüle 7 nebeneinander befinden, vgl. Fig. 6a. Es kann sich aber auch um ein speziell für diesen Zweck hergestelltes Rohr 14 handeln, das durch seine unrunde Gestaltung Platz für den Katheter 5 und die Spinalkanüle 7 bietet, ohne in seiner Gesamtheit einen so großen Querschnitt zu haben wie das Rohr 1, vgl. Fig. 6b.

Die Fig. 7 zeigt den Verlauf der Spinalkanüle 7 neben dem Katheter 5 in einer herkömmlichen Epiduralkanüle 1. Dabei treten Katheter und Spinalkanüle aus der Schlifföffnung 12 des Touhy-Schliffes aus. Dies führt dazu, daß die Spinalkanüle geringfügig gebogen wird.

Fig. 8 zeigt eine andere Anordnung, bei der die Spinalkanüle 7 aus einem Loch 15 im Bereich des Touhy-Schliffes austritt. Bei dieser Anordnung braucht die Spinalkanüle nicht gebogen zu werden. Die Anbringung des Loches 15 bietet sich insbesondere an in Zusammenhang mit dem unrunden Epiduralkanülenrohr 14 nach Fig. 6b.

Eine besonders vorteilhafte Gestaltung des Bestecks ergibt sich dadurch, daß die Spinalkanüle geringgradig vorgekrümmt ist, darüber wird sichergestellt, daß ihre Spitze stets an der Wand der Epiduralkanüle entlang läuft und das Loch 15 sicher trifft. Diese Anordnung hat darüber hinaus auch den Vorteil, daß das Besteck, sofern der Anwender es wünscht, auch in dem Sinne der Anmeldung DE 3 922 406 verwendet werden kann, d.h. zum Setzen der Spinalanästhesie vor dem Schieben des Katheters.

Um ein Eindringen von Gewebe in das Loch 15 zu vermeiden, wird ein profilierter Mandrin verwendet, der das gesamte Lumen der Kanüle verschließt und damit auch das Loch 15. Vom Mandrin ist nur der Ansatz dargestellt, vgl. Fig. 8.

Damit der Anwender auf einfache Weise erkennt, ob die Spinalkanüle 7 die Spitze der Epiduralkanüle 1 erreicht hat, kann auf der Spinalkanüle ein Markierungsring 20 vorgesehen werden, vgl. Fig. 8, oder es kann der Luerkegel 8' des Ansatzes 8 der Spinalkanüle 7 entsprechend verlängert werden, vgl. Fig. 8, in der die Verlängerung des Luerkegels 8' gestrichelt eingezeichnet ist und mit dem Bezugszeichen 8'' versehen ist. Das Eintreten der Markierung 20 oder der Verlängerung 8'' in den Ansatz 2 der Epiduralkanüle zeigt dann an, daß die Spitze der Spinalkanüle die Spitze der Epiduralkanüle erreicht hat.

Der Ansatz 8 und/oder der Luerkegel 8' der Spinalkanüle 7 kann, wie dies in der Fig. 9 im Schnitt schematisch dargestellt ist, auf einer Seite mit einer sich in Kanülenrichtung erstreckenden Einkerbung oder Rinne 22 zur Aufnahme des Epiduralkatheters 5 ausgestattet sein, so daß der Ansatz 8 auch bei bereits eingeschobenem Epiduralkatheter 5 in den Ansatz 2 der Epiduralkanüle 1 leichter eingeschoben werden kann.

Das vordere Ende des Luerkegels 8' des Ansatzes 8 der Spinalkanüle 7 kann auch durch einen Schlitz 24 in zwei Teile 26 und 28 aufgeteilt ausgebildet sein, vgl. Fig. 10, zwischen denen Platz für den Epiduralkatheter 5 belassen ist. Da beide Teile 26 und 28 gegeneinander federnd angeordnet sind, kann der Luerkegel 8' innerhalb einer Strecke von circa 6 mm im Ansatz 2 der Epiduralkanüle 1 an beliebiger Stelle klemmend fixiert werden.

## Patentansprüche

1. Anästhesiebesteck bestehend aus einer Epiduralkanüle (1) mit Touhyschlifföffnung (12), einem Epiduralkatheter (5) und einer Spinalkanüle (7), dadurch gekennzeichnet, daß der Durchmesser, bzw. Querschnitt der Epiduralkanüle (1) so bemessen ist, daß bei darinliegendem Epiduralkatheter (5) noch ausreichend Platz für das Hindurchschieben der Spinalkanüle (7) zwischen der Wandung der Epiduralkanüle (1) und dem Epiduralkatheter (5) besteht.

2. Anästhesiebesteck nach Anspruch 1, dadurch gekennzeichnet, daß das Rohr der Epiduralkanüle nicht rund ist,sondern einseitig eliptisch (14) gestaltet ist, so daß Katheter und Spinalkanüle hindurch passen, der Gesamtquerschnitt des Epiduralkanülenrohres aber kleiner ist als bei einem entsprechenden runden Rohr.

3. Anästhesiebesteck nach Anspruch 1, dadurch gekennzeichnet, daß im Bereich der Spitze der Epiduralkanüle (1), gegenüber der Schlifföffnung (12), ein Loch (15) angeordnet ist, durch das die Spinalkanüle heraustreten kann, ohne daß ihre Spitze gebogen wird.

4. Anästhesiebesteck nach Anspruch 2 oder 3, dadurch gekennzeichnet, daß die Spinalkanüle leicht gekrümmt ist, um ihrer Spitze eine eindeutige Lage im Innenraum der Epiduralkanüle zu geben.

5. Anästhesiebesteck nach Anspruch 3, dadurch gekennzeichnet, daß das Loch (15) exzentrisch der Schlifföffnung (12) gegenüber angeordnet ist.

6. Anästhesiebesteck nach Anspruch 1, dadurch gekennzeichnet, daß am hinteren Ende der Spinalkanüle (7) ein an sich bekannter Ansatz (8) angeordnet ist, in dessen Außenfläche sich eine in Kanülenrichtung erstreckende Rinne oder Einkerbung (22) für den Epiduralkatheter (5) angeordnet ist.

7. Anästhesiebesteck nach Anspruch 6, dadurch gekennzeichnet, daß die Rinne oder Einkerbung (22) an einem am vorderen Ende des Ansatzes (8) vorgesehenen Luerkegel (8') ausgebildet ist.

8. Anästhesiebesteck nach Anspruch 6, dadurch gekennzeichnet, daß der Ansatz (8) an seinem vorderen Ende mit einem Luerkegel (8') versehen ist, der am vorderen Ende durch einen Schlitz (24) in zwei federnde Teile (26, 28) aufgeteilt ist, zwischen denen Platz für den Epiduralkatheter (5) belassen ist.

9. Anästhesiebesteck nach Anspruch 1, dadurch gekennzeichnet, daß die Spinalkanüle (7) und die Epiduralkanüle (1) jeweils mit einem Mandrin versehen sind, der dem lichten Querschnitt der Kanülen (7) und (1) angepaßt ist.

10. Anästhesiebesteck nach Anspruch 9 und 3, dadurch gekennzeichnet, daß der Mandrin profiliert ausgebildet ist und sich bis in das Loch (15) erstreckt und mit seiner Stirnfläche mit der Außenfläche der Epiduralkanüle fluchtet.

11. Anästhesiebesteck nach einem der vorhergehenden An sprüche, dadurch gekennzeichnet, daß auf der Spinalkanüle (7) eine Markierung (20) vorgesehen ist, deren Eintreten in einen Ansatz (2) der Epiduralkanüle (1) anzeigt, daß die Spiralkanülenspitze die Spitze der Epiduralkanüle erreicht hat.

12. Anästhesiebesteck nach einem der Ansprüche 1 bis 10, dadurch gekennzeichnet, daß der Luerkegel (8') der Spinalkanüle (7) eine Verlängerung (8'') aufweist, deren Eintreten in einen Ansatz (2) der Epiduralkanüle (1) anzeigt, daß die Spinalkanülenspitze die Spitze der Epiduralkanüle erreicht hat.

## Claims

1. Anaesthetic equipment comprising an epidural needle (1) provided with a Touhy's ground opening (12), an epidural catheter (5) and a spinal needle (7), characterised in that the diameter or cross-sectional area of the epidural needle (1) is so dimensioned that when the epidural catheter (5) is situated in it there is still enough space for moving through the spinal needle (7) between the wall of the epidural needle (1) and the epidural catheter (5).

2. Anaesthetic equipment according to claim 1, characterised in that the tube of the epidural needle is not round but is on one side elliptical (14) so that the catheter and the spinal needle can pass therethrough, but the total cross-sectional area of the hollow shaft of the epidural needle is smaller that it would be if the tube were round.

3. Anaesthetic equipment according to claim 1, characterised in that in the region of the tip of the epidural needle (1), opposite to the ground opening (12), is situated an opening (15) through which can project the spinal needle without its tip being bent.

4. Anaesthetic equipment according to claim 2 or 3, characterised in that the spinal needle is slightly curved so that its tip has a definite position inside the epidural needle.

5. Anaesthetic equipment according to claim 3, characterised in that the opening (15) is arranged eccentrically with respect to the ground opening (12).

6. Anaesthetic equipment according to claim 1, characterised in that at the rear end of the spinal needle (7) is provided a hub (8) known per se, in whose outer surface is made a notch or groove (22) for the epidural catheter (5), the notch or groove extending in the direction of the needle.

7. Anaesthetic equipment according to claim 6, characterised in that the notch or groove (22) is made on a Luer's cone (8') provided on the front end of the hub (8).

8. Anaesthetic equipment according to claim 6, characterised in that the hub (8) is at its front end provided with a Luer's cone (8') which is subdivided at its front end by a slit (24) into two resilient portions (26,28) between which is left a space for the epidural catheter (5).

9. Anaesthetic equipment according to claim 1, characterised in that the spinal needle (7) and the epidural needle (1) are each provided with a mandrin which is adapted to the inner cross-section of the needles (7) and (1).

10. Anaesthetic equipment according to claim 9 and 3, characterised in that the mandril is profiled and extends up into the opening (15) and its end face is in alignment with the outer surface of the epidural needle.

11. Anaesthetic equipment according to any one of the preceding claims, characterised in that a marking (20) is provided on the spinal needle (7), the entry of the marking into a hub (2) of the epidural needle (1) indicates that the tip of the spinal needle has reached the tip of the epidural needle.

12. Anaesthetic equipment according to any one of claims 1 to 10, characterised in that the Luer's cone (8') of the spinal needle (7) has an extension (8''), whose entry into a hub (2) of the epidural needle (1) indicates that the tip of the spinal needle has reached the tip of the epidural needle.

## Revendications

1. Ensemble pour piqûre d'anesthésie constitué d'une canule épidurale (1) avec une ouverture polie de Touhy (12), un cathéter épidural (5) et une canule spinale (7),
caractérisé en ce que le diamètre ou respectivement la section transversale de la canule épidurale (1) a une dimension telle que, avec le cathéter épidural (5) logé dedans, il existe encore suffisamment d'espace pour le coulissement à travers de la canule spinale (7) entre la paroi de la canule épidurale (1) et le cathéter épidural (5).

2. Ensemble pour piqûre d'anesthésie selon la revendication 1,
caractérisé en ce que le tube de la canule épidurale n'est pas rond mais est formé elliptique (14) d'un côté de sorte que le cathéter et la canule spinale sont en correspondance à travers, mais l'ensemble de la section transversale du tube de la canule épidurale est plus petite que dans le cas d'un tube rond correspondant.

3. Ensemble pour piqûre d'anesthésie selon la revendication 1,
caractérisé en ce que, dans la zone de la pointe de la canule épidurale (1), en face de l'ouverture polie (12), est disposé un orifice (15) à travers lequel la canule spinale peut ressortir sans que sa pointe soit courbée.

4. Ensemble pour piqûre d'anesthésie selon la revendication 2 ou 3,
caractérisé en ce que la canule spinale est légèrement cintrée pour donner à sa pointe une position unique dans le volume intérieur de la canule épidurale.

5. Ensemble pour piqûre d'anesthésie selon la revendication 3,
caractérisé en ce que l'orifice (15) est disposé excentriquement en face de l'ouverture polie (12).

6. Ensemble pour piqûre d'anesthésie selon la revendication 1,
caractérisé en ce que, à l'extrémité arrière de la canule spinale (7), est disposé un appendice (8) connu en soi dans la surface extérieure duquel est disposée une rainure ou entaille (22) s'étendant en direction de la canule, pour le cathéter épidural (5).

7. Ensemble pour piqûre d'anesthésie selon la revendication 6,
caractérisé en ce que la rainure ou entaille (22) est formée sur un cône de Luer (8') disposé à une extrémité antérieure de l'appendice (8).

8. Ensemble pour piqûre d'anesthésie selon la revendication 6,
caractérisé en ce que l'appendice (8) est muni à son extrémité frontale d'un cône de Luer (8') qui est divisé à l'extrémité antérieure, par une fente (24) en deux parties élastiques (26,28) entre lesquelles est laissé un espace pour le cathéter épidural (5).

9. Ensemble pour piqûre d'anesthésie selon la revendication 1,
caractérisé en ce que la canule spinale (7) et la canule épidurale (1) sont munies chacune d'un mandrin qui est adapté à la section transversale intérieure des canules (7) et (1).

10. Ensemble pour piqûre d'anesthésie selon les revendications 9 et 3,
caractérisé en ce que le mandrin est réalisé profilé et s'étend jusque dans l'orifice (15) et est aligné par sa surface frontale avec la surface extérieure de la canule épidurale.

11. Ensemble pour piqûre d'anesthésie selon l'une des revendications précédentes,
caractérisé en ce que, sur la canule spinale (7), est disposé un marquage (20) dont l'entrée dans un appendice (2) de la canule épidurale (1) indique que la pointe de la canule spinale a atteint la pointe de la canule épidurale.

12. Ensemble pour piqûre d'anesthésie selon l'une des revendications 1 à 10,
caractérisé en ce que le cône de Luer (8') de la canule spinale (7) présente un prolongement (8'') dont l'entrée dans un appendice (2) de la canule épidurale (1) indique que la pointe de la canule spinale a atteint la pointe de la canule épidurale.
